(19) Europäisches Patentamt
European Patent Office
Office européen des brevets

(11) EP 1 292 597 B1

(12) FASCICULE DE BREVET EUROPEEN

(45) Date de publication et mention de la délivrance du brevet:
**17.03.2004 Bulletin 2004/12**

(21) Numéro de dépôt: **01947525.0**

(22) Date de dépôt: **19.06.2001**

(51) Int Cl.[7]: **C07D 493/04**, A61K 31/365, A61P 35/00 // (C07D493/04, 317:00), C07D307:00

(86) Numéro de dépôt international:
**PCT/FR2001/001908**

(87) Numéro de publication internationale:
**WO 2001/098307 (27.12.2001 Gazette 2001/52)**

# (54) DERIVES DE PODOPHYLLOTOXINE CARBAMATE ET THIOCARBAMATE, LEUR PROCEDE DE PREPARATION ET LES COMPOSITIONS PHARMACEUTIQUES QUI LES CONTIENNENT

DERIVATE DES CARBAMATES UND THIOCARBAMATES VON PODOPHYLLOTOXIN, IHRE HERSTELLUNGSWEISE UND PHARMAZEUTISCHE ZUSAMMENSETZUNGEN, DIE DIESE ENTHALTEN

CARBAMATE AND THIOCARBAMATE PODOPHYLLOTOXIN DERIVATIVES, PREPARATION METHOD AND PHARMACEUTICAL COMPOSITIONS CONTAINING THEM

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **20.06.2000 FR 0007823**

(43) Date de publication de la demande:
**19.03.2003 Bulletin 2003/12**

(73) Titulaire: **Les Laboratoires Servier**
**92415 Courbevoie Cedex (FR)**

(72) Inventeurs:
- **MONNERET, Claude**
  **F-75012 Paris (FR)**
- **BERTOUNESQUE, Emmanuel**
  **F-75015 Paris (FR)**
- **MERESSE, Philippe**
  **F-59218 Salesches (FR)**
- **ATASSI, Ghanem**
  **F-92210 Saint-Cloud (FR)**
- **PIERRE, Alain**
  **F-78580 Les Alluets le Roi (FR)**
- **HICKMAN, John**
  **F-75017 Paris (FR)**
- **PFEIFFER, Bruno**
  **F-95320 Saint Leu la Forêt (FR)**
- **RENARD, Pierre**
  **F-78150 Le Chesnay (FR)**

(56) Documents cités:
**FR-A- 1 455 540** **JP-A- 1 117 885**

- **DATABASE CHEMABS [en ligne] CHEMICAL ABSTRACTS SERVICE, COLUMBUS, OHIO, US; GREENWALD, R. B. ET AL: "Drug delivery of anticancer agents: water soluble 4-polyethylene glycol derivatives of the lignan, podophyllotoxin" retrieved from STN Database accession no. 132:15517 XP002161322 & J. CONTROLLED RELEASE (1999), 61(3), 281-294,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux dérivés de podophyllotoxine carbamate et thiocarbamate, leur procédé de préparation et les compositions pharmaceutiques qui les contiennent.

Les composés de l'invention constituent des dérivés de la podophyllotoxine, un lignane naturel connu pour son utilité dans le traitement du cancer. D'autres dérivés synthétiques tels que l'étoposide et la téniposide sont utilisés de façon courante comme agent chimiothérapeutique pour le traitement notamment du cancer du poumon à petites cellules. Ces différents composés agissent en inhibant l'activité catalytique de la topoisomérase II.

Diverses modifications ont été réalisées sur ces dérivés, comme celles décrites dans les demandes de brevet JP 948782, WO 97/13776 et US 3,634,459. Néanmoins; les besoins de la thérapeutique anticancéreuse exigent le développement constant de nouveaux agents antitumoraux et cytotoxiques, dans le but d'obtenir des médicaments à la fois plus actifs, plus solubles et mieux tolérés.

Les composés de la présente invention, outre le fait qu'ils soient nouveaux, présentent une activité in-vivo et in-vitro surprenante et supérieure à celle observée jusqu'ici. Ainsi, les composés découverts par la Demanderesse possèdent des propriétés qui les rendent particulièrement utiles pour le traitement des cancers.

Plus particulièrement, la présente invention concerne les composés de formule **(I)** :

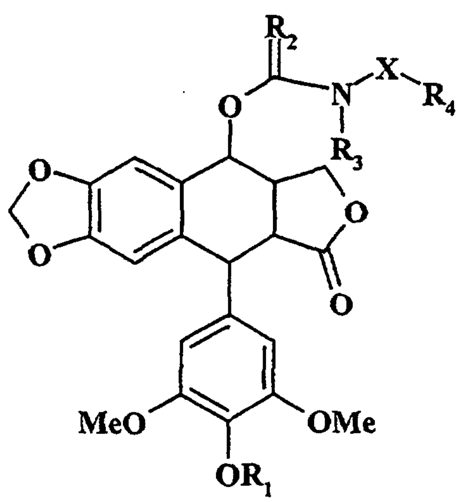

**(I)**

dans laquelle :

- $R_1$ représente un groupement choisi parmi hydrogène, alkyle $(C_1\text{-}C_6)$ linéaire ou ramifié, aryle, arylalkyle $(C_1\text{-}C_6)$ linéaire ou ramifié, hétéroaryle, hétéroarylalkyle $(C_1\text{-}C_6)$ linéaire ou ramifié, alkoxycarbonyle $(C_1\text{-}C_6)$ linéaire ou ramifié, aryloxycarbonyle, arylalkoxycarbonyle $(C_1\text{-}C_6)$ linéaire ou ramifié, hétérocycloalkoxycarbonyle, alkylsulfonyle $(C_1\text{-}C_6)$ linéaire ou ramifié, arylsulfonyle, arylalkylsulfonyle $(C_1\text{-}C_6)$ linéaire ou ramifié, et phosphonique,

- $R_2$ représente un atome d'oxygène ou un atome de soufre,

- $R_3$ représente un atome d'hydrogène, un groupement alkyle $(C_1\text{-}C_6)$ linéaire ou ramifié, cycloalkyle, ou arylalkyle $(C_1\text{-}C_6)$ linéaire ou ramifié,

- X représente un groupement alkylène $(C_1\text{-}C_6)$ linéaire ou ramifié,

- $R_4$ représente un groupement choisi parmi :

  **-**amino éventuellement substitué par un ou deux groupements, identiques ou différents, choisis parmi alkyle $(C_1\text{-}C_6)$ linéaire ou ramifié, aryle et arylalkyle $(C_1\text{-}C_6)$ linéaire ou ramifié,
  **-**un groupement de formule $-N(R_3)-X_1-OR_5$ dans laquelle $R_3$ est tel que défini précédemment, $X_1$ représente un groupement alkylène $(C_1\text{-}C_6)$ linéaire ou ramifié, et $R_5$ représente un groupement choisi parmi atome d'hydrogène, groupement alkyle $(C_1\text{-}C_6)$ linéaire ou ramifié, aryle et arylalkyle $(C_1\text{-}C_6)$ linéaire où ramifié,
  **-**un groupement de formule $-N(R_3)-X_1-NR_6R_7$ dans laquelle $R_3$ et $X_1$ sont tels que définis précédemment, et $R_6$, $R_7$ , identiques ou différents, représentent chacun un groupement choisi parmi atome d'hydrogène, groupement

alkyle ($C_1$-$C_6$) linéaire ou ramifié, aryle et arylalkyle ($C_1$-$C_6$) linéaire ou ramifié,
-hydroxy,
-alkoxy($C_1$-$C_6$) linéaire ou ramifié,
-aryloxy,
-un groupement de formule -O-$X_1$-$OR_5$ dans laquelle $R_5$ et $X_1$ sont tels que définis précédemment,
-et un groupement de formule -O-$X_1$-$NR_6R_7$ dans laquelle $R_6$, $R_7$ et $X_1$ sont tels que définis précédemment,

leurs isomères ainsi que leurs sels d'addition à un acide ou à une base phazmaceutiquement acceptable, étant entendu que :

* *par aryle*, on comprend un groupement choisi parmi phényle, biphényle, naphtyle, dihydronaphtyle, tétrahydronaphtyle, indényle, indanyle, et benzocyclobutyle, chacun de ces groupements comportant éventuellement une ou plusieurs substitutions, identiques ou différentes, choisies parmi halogène, hydroxy, alkyle ($C_1$-$C_6$) linéaire ou ramifié, alkoxy ($C_1$-$C_6$) linéaire ou ramifié, cyano, nitro, amino, alkylamino ($C_1$-$C_6$) linéaire ou ramifié, dialkylamino ($C_1$-$C_6$) linéaire ou ramifié, carboxy, alkoxycarbonyle ($C_1$-$C_6$) linéaire ou ramifié, trihalogénoalkyle ($C_1$-$C_6$) linéaire ou ramifié, alkylcarbonyloxy ($C_1$-$C_6$) linéaire ou ramifié, alkylcarbonyle ($C_1$-$C_6$) linéaire ou ramifié, et aminocarbonyle dans laquelle la partie amino est éventuellement substituée par un ou deux groupements, identiques ou différents, alkyle ($C_1$-$C_6$) linéaire ou ramifié,

* *par hétéroaryle*, on comprend un monocycle aromatique, un bicycle aromatique, ou un bicycle dont l'un des cycles est aromatique et l'autre cycle est partiellement hydrogéné, de 5 à 12 chaînons, comportant au sein du système cyclique un, deux ou trois hétéroatomes choisis parmi oxygène, azote et soufre, ledit hétéroaryle pouvant éventuellement être substitué par les mêmes substitutions que celles décrites dans le cas du groupement aryle,

* *par cycloalkyle*, on comprend un groupement monocyclique ou bicyclique, saturé ou insaturé mais sans caractère aromatique, comportant de 3 à 10 atomes de carbone, étant éventuellement substitué par un ou plusieurs groupements, identiques ou différents, choisis parmi halogène, alkyle ($C_1$-$C_6$) linéaire ou ramifié, trihalogénoalkyle ($C_1$-$C_6$) linéaire ou ramifié, hydroxy, amino, alkylamino ($C_1$-$C_6$) linéaire ou ramifié, et dialkylamino ($C_1$-$C_6$) linéaire ou ramifié,

* *par hétérocycloalkyle*, on comprend un cycloalkyle tel que défini précédemment, comportant au sein du système cyclique, un ou deux hétéroatomes choisis parmi oxygène, azote et soufre, ledit hétérocycloalkyle étant éventuellement substitué par un ou plusieurs substituants tels que ceux décrits dans le cas du groupement cycloalkyle.

Par isomères, on comprend les énantiomères et les diastéréoisomères.
Parmi les acides pharmaceutiquement acceptables, on peut citer à titre non limitatif, les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthane sulfonique, camphorique, etc...
Parmi les bases pharmaceutiquement acceptables, on peut citer à titre non limitatif l'hydroxyde de sodium, l'hydroxyde de potassium, la triéthylamine, la tertbutylamine, etc...
Le substituant $R_1$ préféré selon l'invention est l'atome d'hydrogène.
Le substituant $R_2$ préféré selon l'invention est l'atome d'oxygène.
D'une façon très avantageuse, les composés préférés de l'invention sont les composés de formule (I) dans laquelle X représente un groupement alkylène ($C_2$-$C_4$) linéaire.
Les substituants $R_3$ préférés selon l'invention sont l'atome d'hydrogène et le groupement alkyle ($C_1$-$C_6$) linéaire ou ramifié.
Selon une variante avantageuse de l'invention, les composés préférés de l'invention sont les composés de formule (I) dans laquelle $R_4$ représente un groupement choisi parmi :

- amino substitué par un ou deux groupements, identiques ou différents, alkyle ($C_1$-$C_6$) linéaire ou ramifié,
- un groupement de formule -N($R_3$)-$X_1$-$OR_5$ dans laquelle $R_3$ représente un atome d'hydrogène ou un groupement alkyle ($C_1$-$C_6$) linéaire ou ramifié, $X_1$ et $R_5$ sont tels que définis dans la formule (I),
- et un groupement de formule -O-$X_1$-$OR_5$ dans laquelle $X_1$ et $R_5$ sont tels que définis dans la formule (I).

D'une façon particulièrement avantageuse, les composés préférés de l'invention sont les composés de formule (I) dans laquelle $R_4$ représente un groupement -N($R_3$)-$X_1$-$OR_5$ ou -O-$X_1$-$OR_5$ dans lesquels $R_3$ représente un atome d'hydrogène, $X_1$ représente une chaîne alkylène ($C_2$-$C_4$) linéaire et $R_5$ représente un atome d'hydrogène.
Les composés préférés de l'invention sont le :

- (5*S*,5a*S*,8a*S*,9*R*)-9-(4-hydroxy-3,5-diméthoxyphényl)-8-oxo-5,5a,6,8,8a,9-hexahydrofuro [3',4':6,7]naphtho [2,3-*d*][1,3]dioxol-5-yl 2-(diméthylamino)éthyl(méthyl) carbamate,
- (5*S*,5a*S*,8a*R*,9*R*)-9-(4-hydroxy-3,5-diméthoxyphényl)-8-oxo-5,5a,6,8,8a,9-hexahydrofuro [3',4':6,7]naphtho [2,3-*d*][1,3]dioxol-5-yl 2-(diméthylamino)éthyl(méthyl)carbamate,
- (5*S*,5a*S*,8a*S*,9*R*)-9-(4-hydroxy-3,5-diméthoxyphényl)-8-oxo-5,5a,6,8,8a,9-hexahydrofuro [3',4':6,7]naphtho [2,3-*d*][1,3]dioxol-5-yl 3-(diméthylamino)propylcarbamate,
- (5*S*,5a*S*,8a*S*,9*R*)-9-(4-hydroxy-3,5-diméthoxyphényl)-8-oxo-5,5a,6,8,8a,9-hexahydrofuro [3',4':6,7]naphtho [2,3-*d*][1,3]dioxol-5-yl 2-(2-hydroxyéthoxy)éthylcarbamate,
- (5*S*,5a*S*,8a*S*,9*R*)-9-(4-hydroxy-3,5-diméthoxyphényl)-8-oxo-5,5a,6,8,8a,9-hexahydrofuro [3',4':6,7]naphtho [2,3-*d*][1,3]dioxol-5-yl 2-[(2-hydroxyéthyl)amino]éthylcarbamate,
- (5S,5aS,8aS,9R)-9-(4-hydroxy-3,5-diméthoxyphényl)-8-oxo-5,5a,6,8,8a,9-hexahydrofuro [3',4';6,7]naphto[2,3-d] [1,3]dioxol-5-yl-2-(diméthylamino)éthylcarbamate.

Les isomères ainsi que les sels d'addition à un acide ou à une base pharmaceutiquement acceptable des composés préférés de l'invention font partie intégrante de l'invention.

La présente invention s'étend également au procédé de préparation des composés de formule (I), caractérisé en ce qu'on utilise comme produit de départ un composé de formule **(II)** :

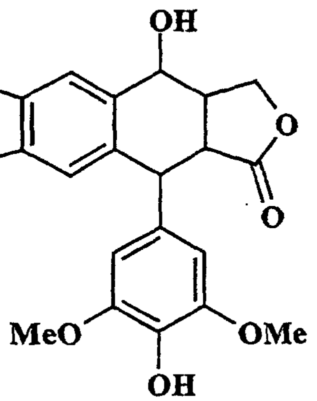

**(II)**

qui est soumis, en condition basique, :

- *soit à l'action d'un composé de formule **(III)** :*

$$R'_1 - X \qquad \textbf{(III)}$$

dans laquelle R'$_1$ représente un groupement choisi parmi alkyle ($C_1$-$C_6$) linéaire ou ramifié, aryle, arylalkyle ($C_1$-$C_6$) linéaire ou ramifié, hétéroaryle, hétéroarylalkyle ($C_1$-$C_6$) linéaire ou ramifié, alkylcarbonyle ($C_1$-$C_6$) linéaire ou ramifié, arylcarbonyle, arylalkylcarbonyle ($C_1$-$C_6$) linéaire ou ramifié, hétérocycloalkylcarbonyle, alkylsulfonyle ($C_1$-$C_6$) linéaire ou ramifié, arylsulfonyle, et arylalkylsulfonyle ($C_1$-$C_6$) linéaire ou ramifié, et X représente un atome d'hydrogène ou d'halogène, pour conduire aux composés de formule **(IV/A)** :

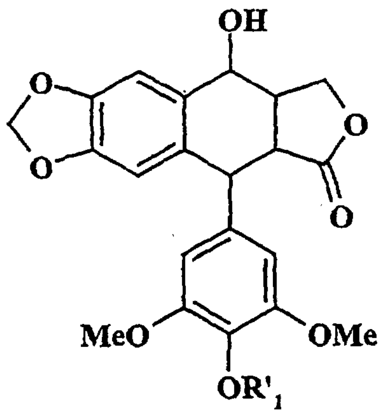

(IV/A)

dans laquelle $R'_1$ est tel que défini précédemment,

- *soit à l'action d'un composé de formule **(V)*** :

**G-L (V)**

dans laquelle G représente un groupement protecteur classique des fonctions hydroxy et L un groupe partant usuel de la chimie organique,
pour conduire aux composés de formule **(IV/B)** :

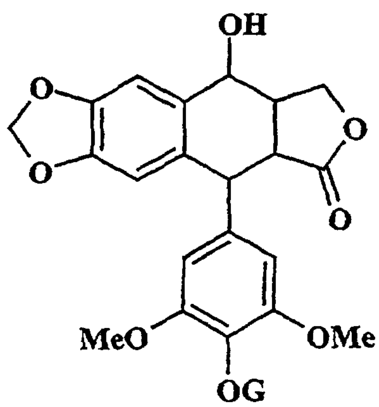

(IV/B)

dans laquelle G est tel que défini précédemment,
l'ensemble des composés de formule (IV/A) et (IV/B) formant les composés de fonnule **(IV)** :

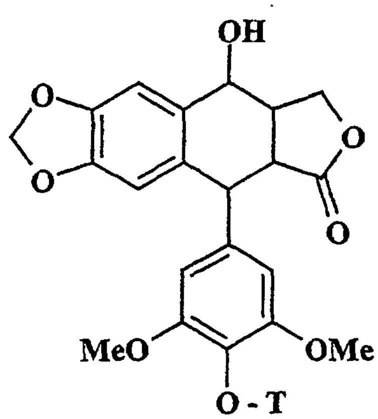

(IV)

dans laquelle T représente un groupement $R'_1$ ou G tel que défini précédemment, composé de formule (IV) que l'on traite en milieu basique par un composé de formule **(VI)** :

$$Ph - O - C(R_2)Cl \qquad \textbf{(VI)}$$

dans laquelle Ph représente un groupement phényle éventuellement substitué et $R_2$ représente un atome d'oxygène ou un atome de soufre,
pour conduire aux composés de formule **(VII)** :

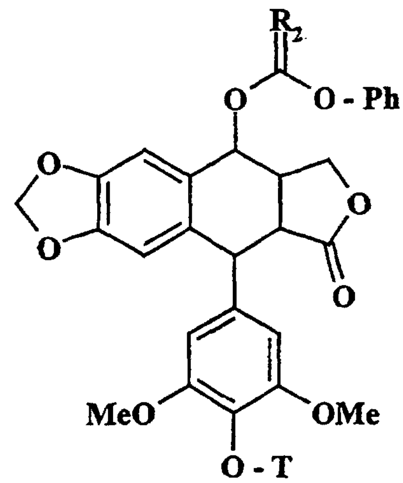

(VII)

dans laquelle T, Ph et $R_2$ sont tels que définis précédemment,
composé de formule (VII), qui est mis à réagir en milieu basique avec un composé de formule **(VIII)** :

$$R_4 - X - NH - R_3 \qquad \textbf{(VIII)}$$

dans laquelle $R_4$, $R_3$ et X sont tels que définis dans la formule (I),
pour conduire respectivement aux composés de formule **(I/a)**, cas particulier des composés de formule (I), ou **(IX)**, selon que T représente un groupement $R'_1$ ou G,

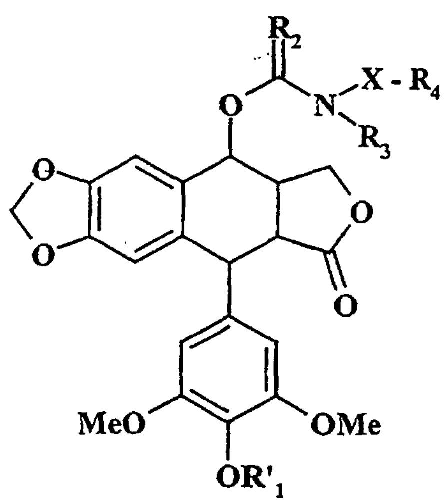

,

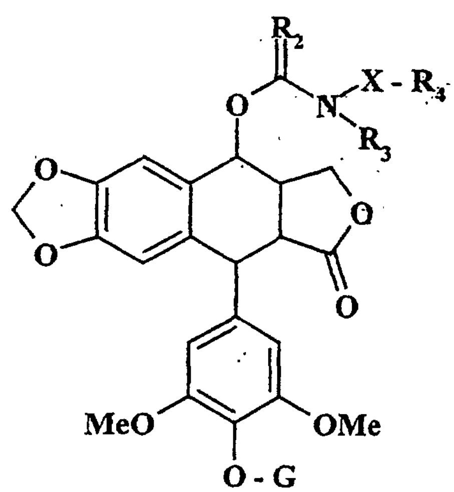

(I/a) (IX)

dans lesquels $R'_1$, G, $R_2$, $R_3$, $R_4$ et X sont tels que définis précédemment,
composés de formule (IX) dont on déprotège la fonction hydroxy selon les méthodes classiques de la chimie organique, pour conduire aux composés de formule **(I/b)**, cas particulier des composés de formule (I) :

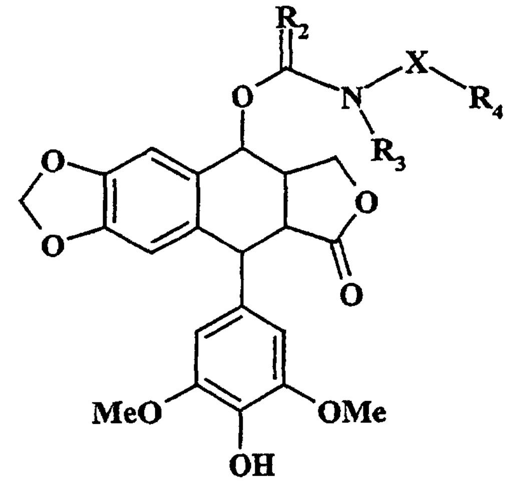

(I/b)

dans laquelle $R_2$, $R_3$, $R_4$ et X sont tels que définis dans la formule (I),
les composés (I/a) et (I/b) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, si on le souhaite, en leurs sels d'addition à un acide ou à une base pharmaceutiquement acceptable.

Les composés de formule (II), (V), (VI) et (VIII) sont soit des composés commerciaux, soit obtenus selon les méthodes classiques de synthèse organique.

Les composés de formule (I) présentent des propriétés antitumorales particulièrement intéressantes. Ils ont une excellent cytotoxicité in vitro sur des lignées cellulaires, issues de tumeurs murines et humaines, et sont actifs in vivo. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses isomères optiques, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux; les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent l'invention mais ne la limitent en aucune façon. Les produits de départ utilisés sont des produits connus ou préparés selon des modes opératoires connus.

**<u>Préparation 1</u> : (5*S*,5a*S*,8a*S*,9*R*)-9-(4-{[(Benzyloxy)carbonyl]oxy}-3,5-diméthoxyphényl)-8-oxo-5,5a,6,8,8a,9-hexahydrofuro[3',4':6,7]naphtho[2,3-d] [1,3]dioxol-5-yl 4-nitrophényl carbonate**

***<u>Stade 1</u> : 4-[(5R,5aS,8aS,9S)-9-Hydroxy-6-oxo-5,5a,6,8,8a,9-hexahydrofuro[3',4':6,7]naphtho[2,3-d][1,3]dioxol-5-yl]-2,6-diméthoxyphényl benzyl carbonate***

A une solution de 0,25 mmol de (5*R*,5a*S*,8a*S*,9*S*)-9-hydroxy-5-(4-hydroxy-3,5-dimethoxyphenyl)-5,8,8a,9-tetrahydrofuro[3',4':6,7]naphtho[2,3-*d*][1,3]dioxol-6(5a*H*)-one dans 10 ml de dichlorométhane anhydre, à 0°C et sous argon, sont additionnées successivement 0,55 mmol de triéthylamine et 0,37 mmol de chloroformate de benzyle. Après 1 heure d'agitation, le milieu réactionnel est lavé à l'eau. La phase organique est ensuite séchée, filtrée puis concentrée

sous pression réduite. Une chromatographie sur gel de silice (cyclohexane/acétate d'éthyle : 60/40) permet d'isoler le produit attendu.

***Stade 2* : (5S,5aS,8aS,9R)-9-(4-{[(Benzyloxy)carbonyl]oxy}-3,5-diméthoxyphényl)-8-oxo-5,5a,6,8,8a,9-hexahydrofuro[3',4':6,7]naphtho[2,3-d][1,3] dioxol-5-yl 4-nitrophényl carbonate**

A une solution de 1,71 mmol de chloroformate de p-nitrophényle dans 5 ml de dichlorométhane anhydre est additionné 0,155 ml de pyridine anhydre. Un précipité blanc se forme dans le milieu réactionnel. Une solution de 0,502 mmol du composé obtenu au stade 1 dans 5 ml de dichlorométhane est ensuite ajoutée, goutte à goutte, sous argon. L'agitation est poursuivie 45 minutes à température ambiante, puis 15 ml d'eau sont versés dans le milieu réactionnel. La phase organique est lavée avec de l'eau, séchée sur $MgSO_4$, filtrée et concentrée sous pression réduite. Une chromatographie sur gel de silice (cyclohexane/acétate d'éthyle : 4/1) permet d'isoler le produit attendu.
***Point de fusion* = 125-130 °C**

**EXEMPLE 1 : (5*S*,5a*S*,8a*S*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl 2-(diméthylamino)éthyl(méthyl)carbamate**

***Stade 1*: 4-[(5R,5aS,8aS,9S)-9-({[[2-(Diméthylamino)éthyl](méthyl) amino]carbonyl}oxy)-6-oxo-5,5a,6,8,8a,9-hexahydrofuro[3',4':6,7] naphtho[2,3-d][1,3]dioxol-5-yl]-2,6-diméthoxyphényl benzyl carbonate**

A une solution de 0,171 mmol du composé de la préparation 1 dans 5 ml de dichlorométhane anhydre sont additionnées successivement, à température ambiante sous argon, 0,228 mmol de *N,N,N'*-triméthyléthylènediamine et 0,228 mmol de triéthylamine. Après 1 heure d'agitation, le milieu réactionnel est hydrolysé. La phase organique est alors lavée avec de l'eau, puis séchée sur $MgSO_4$, filtrée et concentrée sous pression réduite. Une chromatographie sur gel de silice (dichlorométhane/méthanol : 97/3) permet d'isoler le produit attendu.
***Pont de fusion* = 102-103 °C**

***Stade 2* : (5S,5aS,8aS,9R)-9-(4-Hydroxy-3,5-diméthoxyphényl)-8-oxo-5,5a,6,8,8a,9-hexahydrofuro[3',4':6,7]naphtho[2,3-d][1,3]dioxol-5-yl 2-(diméthplamino)éthyl(méthyl)carbamate**

A une solution de 0,138 mmol du composé obtenu au stade 1 dans 8 ml d'acétate d'éthyle est ajouté du charbon palladié à 5% (90,8 mg). Le milieu réactionnel est agité sous atmosphère d'hydrogène (pression atmosphérique) pendant 1 h 45, puis filtré sur célite pour éliminer le catalyseur. Le filtrat est alors concentré sous pression réduite. Le résidu brut est chromatographié sur gel de silice (dichlorométhane/méthanol : 96/4) permettant d'isoler le produit attendu.
***Point de fusion* = 184-186 °C**
***Spectre de masse* (ES/SM) : m/z = 529 $[M + H]^+$, 551 $[M + Na]^+$**

**EXEMPLE 2 : (5*S*,5a*S*,8a*R*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl 2-(diméthylamino)éthyl(méthyl)carbamate**

***Stade 1* : 4-[(5R,5aR,8aS,9S)-9-({[[2-(Diméthylamino)éthyl](méthyl)amino] carbonyl}oxy)-6-oxo-5,5a,6,8,8a,9-hexahydrofuro [3',4':6,7]naphtho [2,3-d][1,3]dioxol-5-yl]-2,6-diméthoxyphényl benzyl carbonate**

A une solution de 0,141 mmol du composé de la préparation 1 dans 4 ml de dichlorométhane anhydre sont additionnées successivement 0,184 mmol de *N,N,N'*-triméthyléthylènediamine et 0,184 mmol de triéthylamine. Après 2 h 30 d'agitation à température ambiante sous argon, on additionne alors, dans le milieu réactionnel, 3 ml de tétrahydrofurane et une solution 1M de fluorure de tétrabutylammonium (0,285 mmol) dans le tétrahydrofurane. Après 2 heures de réaction, 0,3 mmol de fluorure de tétrabutylammonium sont ajouées. L'agitation est poursuivie 3 h 30 et le milieu réactionnel est versé dans de l'eau (15 ml) et dilué avec du dichlorométhane (10 ml). La phase aqueuse est extraite avec du dichlorométhane et les phases organiques réunies sont lavées avec de l'eau et une solution aqueuse saturée en NaCl, puis séchées sur $MgSO_4$, filtrées et concentrées sous pression réduite. Une chromatographie sur gel de silice (dichlorométhane/méthanol : 97/3) permet d'isoler le produit attendu.
***Spectre de masse* ($DIC/NH_3$) : m/z = 663 $[M + H]^+$**

***Stade 2 :*** ***(5S,5aS,8aR,9R)-9-(4-Hydroxy-3,5-diméthoxyphényl)-8-oxo-5,5a,6,8,8a,9-hexahydrofuro[3',4':6,7]naphtho[2,3-d][1,3]dioxol-5-yl 2-(diméthylamino)éthyl(méthyl)carbamate***

Le produit est obtenu selon le procédé du stade 2 de l'exemple 1 en utilisant comme substrat le composé obtenu au stade 1 précédent.
***Spectre de masse (DIC/$NH_3$) : m/z = 529 [M + H]$^+$***

**EXEMPLE 3 : (5*S*,5a*S*,8a*S*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl 3-(diméthylamino)propylcarbamate**

Le produit est obtenu selon le procédé de l'exemple 1, des stades 1 à 2, en utilisant comme réactif au stade 1 la diméthylaminopropylamine comme réactif à la place de la *N,N,N'*-triméthyléthylènediamine.
***$[\alpha]_D$ ($CHCl_3$) =-23°***
***Spectre de masse (DIC/$NH_3$) : m/z = 529 [M+H]$^+$***

**EXEMPLE 4 : (5*S*,5a*S*,8a*S*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl 2-(2-hydroxyéthoxy)éthylcarbamate**

Le produit est obtenu selon le procédé de l'exemple 1, des stades 1 à 2, en utilisant comme réactif au stade 1 le 2-aminoéthoxyéthanol comme réactif à la place de la *N,N,N'*-triméthyléthylènediamine.
***Spectre de masse (DIC/$NH_3$) : m/z = 683 [M+$NH_4$]$^+$***

**EXEMPLE 5 : (5*S*,5a*S*,8a*S*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl 2-[(2-hydroxyéthyl)amino]éthylcarbamate**

Le produit est obtenu selon le procédé de l'exemple 1, des stades 1 à 2, en utilisant comme réactif au stade 1 le 2-aminoéthylaminoéthanol comme réactif à la place de la *N,N,N'*-triméthyléthylènediamine.

**EXEMPLE 6 : (5*S*,5a*S*,8a*S*,9*R*)-9-(4-Hydroxy-3,5-diméthoxyphényl)-8-oxo-5,5a,6,8,8a,9-hexabydrofuro[3',4':6,7]naphto[2,3-*d*][1,3]dioxol-5-yl-2-(diméthylamino)éthylcarbamate**

Le produit est obtenu selon le procédé de l'exemple 1, des stades 1 à 2, en utilisant comme réactif au stade 1 la diméthylaminoéthylamine comme réactif à la place de la *N,N,N'*-triméthyléthylènediamine.

## *ETUDE PHARMACOLOGIQUE DES COMPOSES DE L'INVENTION*

**EXEMPLE 7 : Activité in vitro**

Les composés de l'invention ont été testés sur différentes lignées cellulaires murines ou humaines:

- la leucémie murine L1210,
- le carcinome épidermoïde A-431,
- le carcinome pulmonaire non à petites cellules A549,
- le carcinome pulmonaire à petites cellules H69,
- le carcinome du colon HT29,
- le carcinome épidermoïde buccal KB-3-1.

Les cellules sont cultivées dans du milieu de culture RPMI 1640 complet contenant 10 % de sérum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 heures ou 96 heures. Le nombre de cellules viables est ensuite quantifié par un essai colorimétrique, le Microculture Tetrazolium Assay (J. Carmichael et coll., Cancer Res.; 47, 936-942, (1987)). Les résultats sont exprimés en $IC_{50}$, concentration en cytotoxique qui inhibe à 50 % la prolifération des cellules traitées.
Lors de ces tests, le composé de l'exemple 6 présente les $IC_{50}$ suivantes :

| | IC$_{50}$ (µM) | | | | | |
|---|---|---|---|---|---|---|
| | L1210 | A-431 | A549 | H69 | HT29 | KB-3-1 |
| Exemple 6 | 0,038 | 0,071 | 0,046 | 0,096 | 0,121 | 0,085 |

**EXEMPLE 8 : Action sur le cycle cellulaire**

Les cellules L1210 sont incubées pendant 21 heures à 37°C en présence de différentes concentrations en produit testés. Les cellules sont ensuite fixées par de l'éthanol à 70 % (v/v), lavées deux fois dans du PBS et incubées 30 minutes à 20°C dans du PBS contenant 100 µg/ml de RNAse et 50 µg/ml d'iodure de propidium. Les résultats sont exprimés en pourcentage des cellules accumulées en phase G2+M après 21 heures par rapport au témoin (témoin : 20 %). Le composé de l'exemple 6 induit une accumulation à 86 % des cellules en phase G2+M après 21 heures à une concentration de 100 nM..

**EXEMPLE 9 : Activité in vivo**

** Activité antitumorale des composés sur la leucémie P388 :*

La lignée P388 (leucémie murine) a été fournie par le National Cancer Institute (Frederick, USA). Les cellules tumorales ($10^6$ celules) ont été inoculées au jour 0 dans la cavité péritonéale de souris BDF1 femelles (Iffa-Credo, France) pesant de 18 à 20 g (groupes de 6 animaux). Les produits ont été administrés au jour 1 par voie intraveineuse. L'activité antitumorale est exprimée en % de T/C :

$$\% \text{ T/C} = \frac{\text{Temps de survie médian des animaux traités}}{\text{Temps de survie médian des animaux contrôles}} \times 100$$

A titre indicatif, le composé de l'exemple 6 est actif sur la leucémie P388 et il induit un T/C de 203 % à 6,25 mg/kg.

** Activité antitumorale des composés sur le carcinome pulmonaire de Lewis :*

Le carcinome pulmonaire de Lewis (LLC) est une tumeur solide qui est apparue spontanément dans le poumon d'une souris C57B1/6 et qui est maintenue par transplantations successives de fragments en s.c. Le LLC a été obtenu auprès de la Division of Cancer Treatment, Tumor Repository, National Cancer Institute, Frederick, MD, USA. Les souris âgées de 4 à 6 semaines ont été obtenues chez Iffa Credo (Lyon, France), elles pesaient 20 à 22 g au début de l'expérience.
Le premier jour de l'expérience (J0), les tumeurs sont prélevées et découpées en fragments d'environ 30 mg qui sont immédiatement greffés en s.c. dans des souris B6D2F1. Les souris implantées sont ensuite randomisées dans les groupes traités et le groupe témoin (7 animaux par groupe).
Le paramètre utilisé pour évaluer l'activité antitumorale est l'augmentation du temps de survie. De plus, le nombre d'animaux survivants dans chaque groupe est répertorié à la fin de l'expérience, au jour 90.
Les produits ont été administrés selon le schéma décrit ci-dessous. Les résultats sont exprimés en % de T/C, calculé de la même façon que dans le test précédent :

| *Produit* | *Dose (mg/kg)* | *Voie* | *Schéma* | *T/C %* | *Survivants J90* |
|---|---|---|---|---|---|
| **Exemple 1** | 25 | i.v. | J 4, 8, 12 | 165 | 1/7 |
| **Exemple 6** | 3,12 | i.v. | J 4, 8, 12 | 168 | 1/7 |

**EXEMPLE 10 : Composition pharmaceutique : soluté injectable**

| | |
|---|---|
| Composé de l'exemple | 10 mg |

(suite)

| | |
|---|---|
| Eau distillée pour préparations injectables | 25 ml |

## Revendications

**1.** Composés de formule (I) :

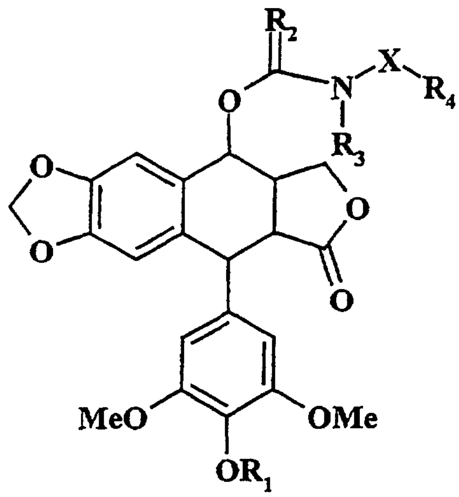

(I)

dans laquelle :

$R_1$ représente un groupement choisi parmi hydrogène, alkyle ($C_1$-$C_6$) linéaire ou ramifié, aryle, arylalkyle ($C_1$-$C_6$) linéaire ou ramifié, hétéroaryle, hétéroarylalkyle ($C_1$-$C_6$) linéaire ou ramifié, alkoxycarbonyle ($C_1$-$C_6$) linéaire ou ramifié, aryloxycarbonyle, arylalkoxycarbonyle ($C_1$-$C_6$) linéaire ou ramifié, hétérocycloalkoxycarbonyle, alkylsulfonyle ($C_1$-$C_6$) linéaire ou ramifié, arylsulfonyle, arylalkylsulfonyle ($C_1$-$C_6$) linéaire ou ramifié, et phosphonique,

$R_2$ représente un atome d'oxygène ou un atome de soufre,

$R_3$ représente un atome d'hydrogène, un groupement alkyle ($C_1$-$C_6$) linéaire ou ramifié, cycloalkyle, ou arylalkyle ($C_1$-$C_6$) linéaire ou ramifié,

X représente un groupement alkylène ($C_1$-$C_6$) linéaire ou ramifié,

$R_4$ représente un groupement choisi parmi :

- amino éventuellement substitué par un ou deux groupements, identiques ou différents, choisis parmi alkyle ($C_1$-$C_6$) linéaire ou ramifié, aryle et arylalkyle ($C_1$-$C_6$) linéaire ou ramifié,
- un groupement de formule $-N(R_3)-X_1-OR_5$ dans laquelle $R_3$ est tel que défini précédemment, $X_1$ représente un groupement alkylène ($C_1$-$C_6$) linéaire ou ramifié, et $R_5$ représente un groupement choisi parmi atome d'hydrogène, groupement alkyle ($C_1$-$C_6$) linéaire ou ramifié, aryle et arylalkyle ($C_1$-$C_6$) linéaire ou ramifié,
- un groupement de formule $-N(R_3)-X_1-NR_6R_7$ dans laquelle $R_3$ et $X_1$ sont tels que définis précédemment, et $R_6$, $R_7$ , identiques ou différents, représentent chacun un groupement choisi parmi atome d'hydrogène, groupement alkyle ($C_1$-$C_6$) linéaire ou ramifié, aryle et arylalkyle ($C_1$-$C_6$) linéaire ou ramifié,
- hydroxy,
- alkoxy ($C_1$-$C_6$) linéaire ou ramifié,
- aryloxy,
- un groupement de formule $-O-X_1-OR_5$ dans laquelle $R_5$ et $X_1$ sont tels que définis précédemment,
- et un groupement de formule $-O-X_1-NR_6R_7$ dans laquelle $R_6$, $R_7$ et $X_1$ sont tels que définis précédemment,

leurs isomères ainsi que leurs sels d'addition à un acide ou à une base pharmaceutiquement acceptable, étant entendu que :

- * *par aryle*, on comprend un groupement choisi parmi phényle, biphényle, naphtyle, dihydronaphtyle, tétrahydronaphtyle, indényle, indanyle, et benzocyclobutyle, chacun de ces groupements comportant éventuellement une ou plusieurs substitutions, identiques ou différentes, choisies parmi halogène, hydroxy, alkyle ($C_1$-$C_6$) linéaire ou ramifié, alkoxy ($C_1$-$C_6$) linéaire ou ramifié, cyano, nitro, amino, alkylamino ($C_1$-$C_6$) linéaire ou ramifié, dialkylamino ($C_1$-$C_6$) linéaire ou ramifié, carboxy, alkoxycarbonyle ($C_1$-$C_6$) linéaire ou ramifié, trihalogénoalkyle ($C_1$-$C_6$) linéaire ou ramifié, alkylcarbonyloxy ($C_1$-$C_6$) linéaire ou ramifié, alkylcarbonyle ($C_1$-$C_6$) linéaire ou ramifié, et aminocarbonyle dans laquelle la partie amino est éventuellement substituée par un ou deux groupements, identiques ou différents, alkyle ($C_1$-$C_6$) linéaire ou ramifié,
- * *par hétéroaryle*, on comprend un monocycle aromatique, un bicycle aromatique, ou un bicycle dont l'un des cycles est aromatique et l'autre cycle est partiellement hydrogéné, de 5 à 12 chaînons, comportant au sein du système cyclique un, deux ou trois hétéroatomes choisis parmi oxygène, azote et soufre, ledit hétéroaryle pouvant éventuellement être substitué par les mêmes substitutions que celles décrites dans le cas du groupement aryle,
- * *par cycloalkyle*, on comprend un groupement monocyclique ou bicyclique, saturé ou insaturé mais sans caractère aromatique, comportant de 3 à 10 atomes de carbone, étant éventuellement substitué par un ou plusieurs groupements, identiques ou différents, choisis parmi halogène, alkyle ($C_1$-$C_6$) linéaire ou ramifié, trihalogénoalkyle ($C_1$-$C_6$) linéaire ou ramifié, hydroxy, amino, alkylamino ($C_1$-$C_6$) linéaire ou ramifié, et dialkylamino ($C_1$-$C_6$) linéaire ou ramifié,
- * *par hétérocycloalkyle*, on comprend un cycloalkyle tel que défini précédemment, comportant au sein du système cyclique, un ou deux hétéroatomes choisis parmi oxygène, azote et soufre, ledit hétérocycloalkyle étant éventuellement substitué par un ou plusieurs substituants tels que ceux décrits dans le cas du groupement cycloalkyle,
- * *par isomères,* on comprend les énantiomères et les diastéréoisomères.

2. Composés de formule (I) selon la revendication 1 **caractérisés en ce que** $R_1$ représente un atome d'hydrogène , leurs isomères ainsi que leurs sels d'addition à un acide ou à une base pharmaceutiquement acceptable.

3. Composés de formule (I) selon la revendication 1 **caractérisés en ce que** $R_2$ représente un atome d'oxygène, leurs isomères ainsi que leurs sels d'addition à un acide ou à une base pharmaceutiquement acceptable.

4. Composés de formule (I) selon la revendication 1 **caractérisés en ce que** X représente un groupement alkylène ($C_2$-$C_4$) linéaire, leurs isomères ainsi que leurs sels d'addition à un acide ou à une base pharmaceutiquement acceptable.

5. Composés de formule (I) selon la revendication 1 **caractérisés en ce que** $R_3$ représente un atome d'hydrogène ou un groupement alkyle ($C_1$-$C_6$) linéaire ou ramifié, leurs isomères ainsi que leurs sels d'addition à un acide ou à une base phannaceutiquement acceptable.

6. Composés de formule (I) selon la revendication 1 **caractérisés en ce que** $R_4$ représente un groupement choisi parmi :

   - amino substitué par un ou deux groupements, identiques ou différents, alkyle ($C_1$-$C_6$) linéaire ou ramifié,
   - un groupement de formule $-N(R_3)-X_1-OR_5$ dans laquelle $R_3$ représente un atome d'hydrogène ou un groupement alkyle ($C_1$-$C_6$) linéaire ou ramifié, $X_1$ et $R_5$ sont tels que définis dans la formule (I),
   - et un groupement de formule $-O-X_1-OR_5$ dans laquelle $X_1$ et $R_5$ sont tels que définis dans la formule (I),

   leurs isomères ainsi que leurs sels d'addition à un acide ou à une base pharmaceutiquement acceptable.

7. Composés de formule (I) selon l'une quelconque des revendications 1 ou 6 **caractérisés en ce que** $R_4$ représente un groupement $-N(R_3)-X_1-OR_5$ ou $-O-X_1-OR_5$ dans lesquels $R_3$ représente un atome d'hydrogène, $X_1$ représente une chaîne alkylène ($C_2$-$C_4$) linéaire et $R_5$ représente un atome d'hydrogène, leurs isomères ainsi que leurs sels d'addition à un acide ou à une base pharmaceutiquement acceptable.

**8.** Composés de formule (I) selon la revendication 1 qui sont le :

- (5*S*,5a*S*,8a*S*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl 2-(diméthylamino)éthyl(méthyl) carbamate,
- (5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl 2-(diméthylamino)éthyl (méthyl)carbamate,
- (5*S*,5a*S*,8a*S*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl 3-(diméthylamino)propylcarbamate,
- (5*S*,5a*S*,8a*S*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl 2-(2-hydroxyéthoxy) éthylcarbamate,
- (5*S*,5a*S*,8a*S*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl 2-[(2-hydroxyéthyl)amino] éthylcarbamate,
- et (5*S*,5a*S*,8a*S*,9*R*)-9-(4-hydroxy-3,5-diméthoxyphényl)-8-oxo-5,5a,6,8,8a,9-hexahydrofuro[3',4':6,7]naphto [2,3-d][1,3]dioxol-5-yl-2-(diméthylamino)éthyl-carbamate, ainsi que leurs isomères ainsi que leurs sels d'addition à un acide ou à une base pharmaceutiquement acceptable.

**9.** Procédé de préparation des composés de formule (I), **caractérisé en ce qu'**on utilise comme produit de départ un composé de formule **(II)** :

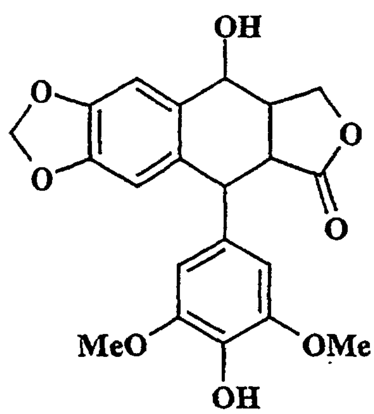

**(II)**

qui est soumis, en condition basique, :

- *soit à l'action d'un composé de formule **(III)** :*

$$R'_1 - X \qquad \textbf{(III)}$$

dans laquelle $R'_1$ représente un groupement choisi parmi alkyle ($C_1$-$C_6$) linéaire ou ramifié, aryle, arylalkyle ($C_1$-$C_6$) linéaire ou ramifié, hétéroaryle, hétéroarylalkyle ($C_1$-$C_6$) linéaire ou ramifié, alkylcarbonyle ($C_1$-$C_6$) linéaire ou ramifié, arylcarbonyle, arylalkylcarbonyle ($C_1$-$C_6$) linéaire ou ramifié, hétérocycloalkylcarbonyle, alkylsulfonyle ($C_1$-$C_6$) linéaire ou ramifié, arylsulfonyle, et arylalkylsulfonyle ($C_1$-$C_6$) linéaire ou ramifié, et X représente un atome d'hydrogène ou d'halogène, pour conduire aux composés de formule **(IV/A)** :

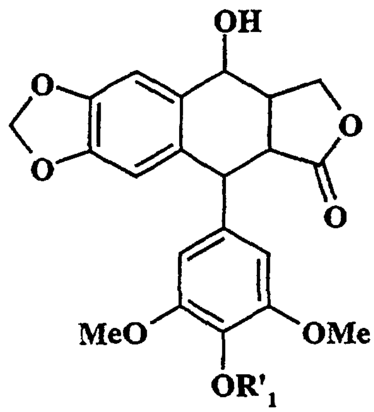

(IV/A)

dans laquelle R'$_1$ est tel que défini précédemment,

- *soit à l'action d'un composé de formule **(V)** :*

**G-L (V)**

dans laquelle G représente un groupement protecteur classique des fonctions hydroxy et L un groupe partant usuel de la chimie organique,
pour conduire aux composés de formule **(IV/B)** :

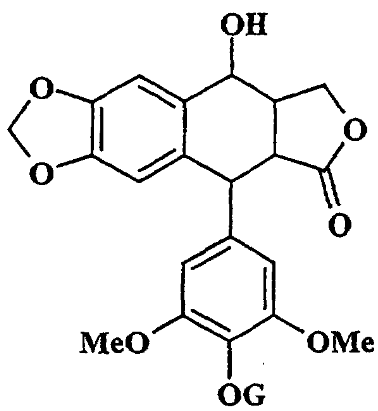

(IV/B)

dans laquelle G est tel que défini précédemment,
l'ensemble des composés de formule (IV/A) et (IV/B) formant les composés de formule **(IV)** :

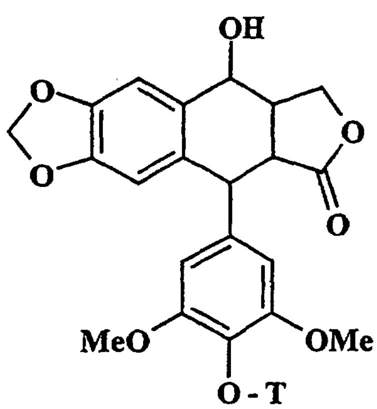

(IV)

dans laquelle T représente un groupement R'$_1$ ou G tel que défini précédemment, composé de formule (IV) que l'on traite en milieu basique par un composé de formule **(VI)** :

$$Ph - O - C(R_2)Cl \qquad (VI)$$

dans laquelle Ph représente un groupement phényle éventuellement substitué et $R_2$ représente un atome d'oxygène ou un atome de soufre,
pour conduire aux composés de formule **(VII)** :

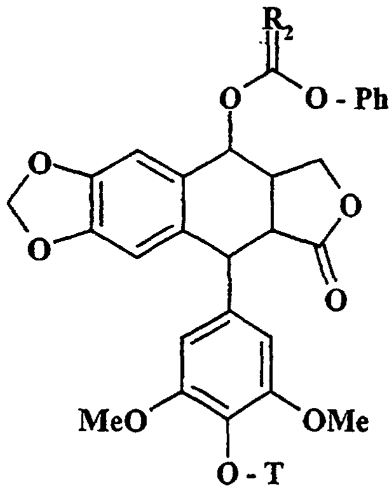

**(VII)**

dans laquelle T, Ph et $R_2$ sont tels que définis précédemment,
composé de formule (VII), qui est mis à réagir en milieu basique avec un composé de formule **(VIII)** :

$$R_4 - X - NH - R_3 \qquad (VIII)$$

dans laquelle $R_4$, $R_3$ et X sont tels que définis dans la formule (I),
pour conduire respectivement aux composés de formule **(I/a)**, cas particulier des composés de formule (I), ou **(IX)**, selon que T représente un groupement R'$_1$ ou G,

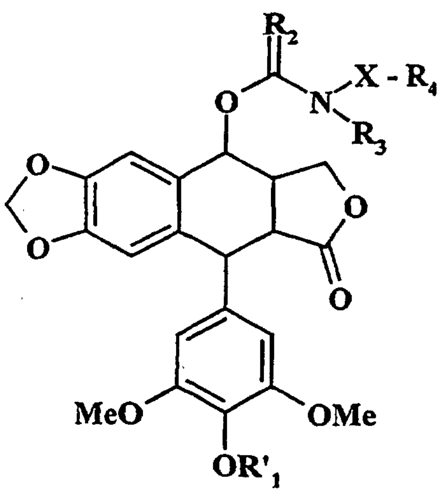

,

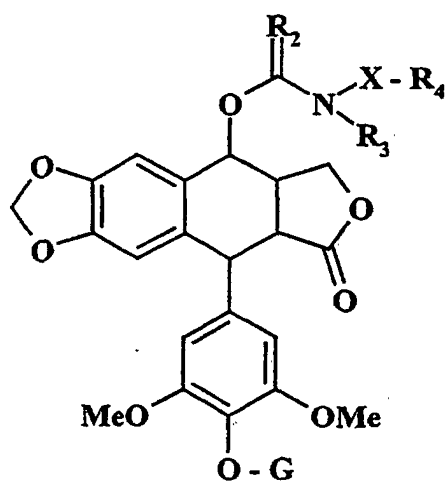

**(I/a)** **(IX)**

dans lesquels R'$_1$, G, $R_2$, $R_3$, $R_4$ et X sont tels que définis précédemment,

composés de formule (IX) dont on déprotège la fonction hydroxy selon les méthodes classiques de la chimie organique, pour conduire aux composés de formule **(I/b)**, cas particulier des composés de formule (I) :

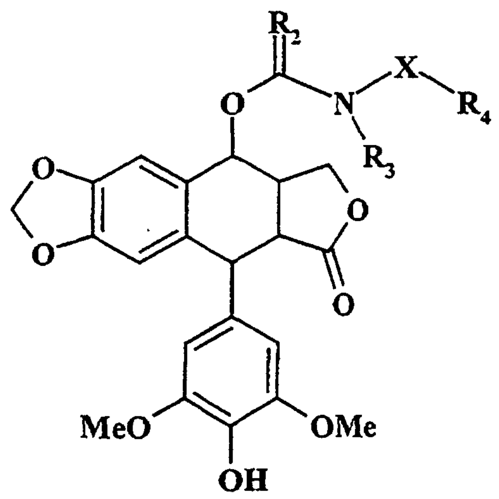

(I/b)

dans laquelle $R_2$, $R_3$, $R_4$ et X sont tels que définis dans la formule (I),
les composés (I/a) et (I/b) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, si on le souhaite, en leurs sels d'addition à un acide ou à une base pharmaceutiquement acceptable.

**10.** Compositions pharmaceutiques contenant comme principe actif au moins un composé selon l'une quelconque des revendications 1 à 8, seul ou en combinaison avec un ou plusieurs excipients ou véhicules inertes, non toxiques, pharmaceutiquement acceptables.

**11.** Compositions pharmaceutiques selon la revendication 10 contenant au moins un principe actif selon l'une quelconque des revendications 1 à 8, utiles dans le traitement des cancers.

## Claims

**1.** Compounds of formula (I) :

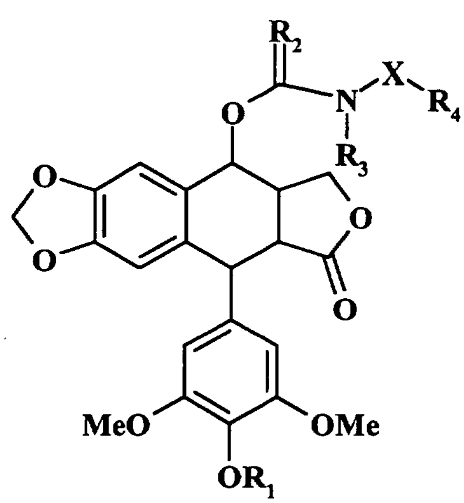

(I)

wherein:

$R_1$ represents a group selected from hydrogen, linear or branched $(C_1-C_6)$alkyl, aryl, aryl-$(C_1-C_6)$alkyl in which the alkyl moiety may be linear or branched, heteroaryl, heteroaryl-$(C_1-C_6)$alkyl in which the alkyl moiety may be linear or branched, linear or branched $(C_1-C_6)$alkoxycarbonyl, aryloxycarbonyl, aryl-$(C_1-C_6)$alkoxycarbonyl in which the alkoxy moiety may be linear or branched, heterocycloalkoxycarbonyl, linear or branched $(C_1-C_6)$alkylsulfonyl, arylsulfonyl, aryl-$(C_1-C_6)$alkylsulfonyl in which the alkyl moiety may be linear or branched, and phosphono,

$R_2$ represents an oxygen atom or a sulphur atom,

$R_3$ represents a hydrogen atom, a linear or branched $(C_1-C_6)$alkyl group, a cycloalkyl group or an aryl-$(C_1-C_6)$alkyl group in which the alkyl moiety may be linear or branched,

X represents a linear or branched $(C_1-C_6)$alkylene group,

$R_4$ represents a group selected from :

- amino optionally substituted by one or two identical or different groups selected from linear or branched $(C_1-C_6)$alkyl, aryl and aryl-$(C_1-C_6)$alkyl in which the alkyl moiety may be linear or branched,
- a group of formula $-N(R_3)-X_1-OR_5$ wherein $R_3$ is as defined hereinbefore, $X_1$ represents a linear or branched $(C_1-C_6)$alkylene group and $R_5$ represents a group selected from hydrogen, linear or branched $(C_1-C_6)$alkyl, aryl and aryl-$(C_1-C_6)$alkyl in which the alkyl moiety may be linear or branched,
- a group of formula $-N(R_3)-X_1-NR_6R_7$ wherein $R_3$ and $X_1$ are as defined hereinbefore and $R_6$ and $R_7$, which may be identical or different, each represents a group selected from hydrogen, linear or branched $(C_1-C_6)$alkyl, aryl and aryl-$(C_1-C_6)$alkyl in which the alkyl moiety may be linear or branched,
- hydroxy,
- linear or branched $(C_1-C_6)$alkoxy,
- aryloxy,
- a group of formula $-O-X_1-OR_5$ wherein $R_5$ and $X_1$ are as defined hereinbefore,
- and a group of formula $-O-X_1-NR_6R_7$ wherein $R_6$, $R_7$ and $X_1$ are as defined hereinbefore,

their isomers, and addition salts thereof with a pharmaceutically acceptable acid or base,
wherein :

* *aryl* denotes a group selected from phenyl, biphenyl, naphthyl, dihydronaphthyl, tetrahydronaphthyl, indenyl, indanyl and benzocyclobutyl, each of those groups optionally having one or more identical or different substituents selected from halogen, hydroxy, linear or branched $(C_1-C_6)$alkyl, linear or branched $(C_1-C_6)$alkoxy, cyano, nitro, amino, linear or branched $(C_1-C_6)$alkylamino, linear or branched di-$(C_1-C_6)$alkylamino, carboxy, linear or branched $(C_1-C_6)$alkoxycarbonyl, linear or branched $(C_1-C_6)$trihaloalkyl, linear or branched $(C_1-C_6)$alkylcarbonyloxy, linear or branched $(C_1-C_6)$alkylcarbonyl, and aminocarbonyl in which the amino moiety is optionally substituted by one or two identical or different linear or branched $(C_1-C_6)$alkyl groups,
* *heteroaryl* denotes an aromatic monocyclic group, an aromatic bicyclic group, or a bicyclic group in which one of the rings is aromatic and the other ring is partially hydrogenated, having from 5 to 12 ring members, containing in the ring system one, two or three hetero atoms selected from oxygen, nitrogen and sulphur, it being possible for the said heteroaryl optionally to be substituted by the same substituents as those decribed in the case of the aryl group,
* *cycloalkyl* denotes a monocyclic or bicyclic group that is saturated or unsaturated, but not of aromatic character, that contains from 3 to 10 carbon atoms and is optionally substituted by one or more identical or different groups selected from halogen, linear or branched $(C_1-C_6)$alkyl, linear or branched $(C_1-C_6)$trihaloalkyl, hydroxy, amino, linear or branched $(C_1-C_6)$alkylamino and linear or branched di-$(C_1-C_6)$alkylamino,
* *heterocycloalkyl* denotes a cycloalkyl group as defined above containing in the ring system one or two hetero atoms selected from oxygen, nitrogen and sulphur, the said heterocycloalkyl being optionally substituted by one or more substituents such as those described in the case of the cycloalkyl group.
* *isomers* is to be understood as meaning the enantiomers and the diastereoisomers.

**2.** Compounds of formula (I) according to claim 1, **characterised in that** $R_1$ represents a hydrogen atom, their isomers, and also addition salts thereof with a pharmaceutically acceptable acid or base.

**3.** Compounds of formula (I) according to claim 1, **characterised in that** $R_2$ represents an oxygen atom, their isomers,

and also addition salts thereof with a pharmaceutically acceptable acid or base.

**4.** Compounds of formula (I) according to claim 1, **characterised in that** X represents a linear ($C_2$-$C_4$)alkylene group, their isomers, and also addition salts thereof with a pharmaceutically acceptable acid or base.

**5.** Compounds of formula (I) according to claim 1, **characterised in that** $R_3$ represents a hydrogen atom or a linear or branched ($C_1$-$C_6$)alkyl group, their isomers, and also addition salts thereof with a pharmaceutically acceptable acid or base.

**6.** Compounds of formula (I) according to claim 1, **characterised in that** $R_4$ represents a group selected from :

- amino substituted by one or two identical or different linear or branched ($C_1$-$C_6$)alkyl groups,
- a group of formula -N($R_3$)-$X_1$-$OR_5$ wherein $R_3$ represents a hydrogen atom or a linear or branched ($C_1$-$C_6$) alkyl group and $X_1$ and $R_5$ are as defined for formula (I),
- and a group of formula -O-$X_1$-$OR_5$ wherein $X_1$ and $R_5$ are as defined for formula (I), their isomers, and also addition salts thereof with a pharmaceutically acceptable acid or base.

**7.** Compounds of formula (I) according to either claim 1 or claim 6, **characterised in that** $R_4$ represents a group -N ($R_3$)-$X_1$-$OR_5$ or -O-$X_1$-$OR_5$ wherein $R_3$ represents a hydrogen atom, $X_1$ represents a linear ($C_2$-$C_4$)alkylene chain and $R_5$ represents a hydrogen atom, their isomers, and also addition salts thereof with a pharmaceutically acceptable acid or base.

**8.** Compounds of formula (I) according to claim 1 that are :

- (5*S*,5a*S*,8a*S*,9*R*)-9-(4-hydroxy-3,5-dimethoxyphenyl)-8-oxo-5,5a,6,8,8a,9-hexahydrofuro[3',4':6,7]naphtho [2,3-*d*][1,3]dioxol-5-yl 2-(dimethylamino)ethyl(methyl)-carbamate,
- (5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho [2,3-*d*][1,3]dioxol-5-yl 2-(dimethylamino)ethyl(methyl)-carbamate,
- (5*S*,5a*S*,8a*S*,9*R*)-9-(4-hydroxy-3,5-dimethoxyphenyl)-8-oxo-5,5a,6,8,8a,9-hexahydrofuro[3',4':6,7]naphtho [2,3-*d*][1,3]dioxol-5-yl 3-(dimethylamino)propylcarbamate,
- (5*S*,5a*S*,8a*S*,9*R*)-9-(4-hydroxy-3,5-dimethoxyphenyl)-8-oxo-5,5a,6,8,8a,9-hexahydrofuro[3',4':6,7]naphtho [2,3-*d*][1,3]dioxol-5-yl 2-(2-hydroxyethoxy)ethylcarbamate,
- (5*S*,5a*S*,8a*S*,9*R*)-9-(4-hydroxy-3,5-dimethoxyphenyl)-8-oxo-5,5a,6,8,8a,9-hexahydrofuro[3',4':6,7]naphtho [2,3-*d*][1,3]dioxol-5-yl 2-[(2-hydroxyethyl)amino]ethylcarbamate,
- and (5S,5aS,8aS,9R)-9-(4-hydroxy-3,5-dimethoxyphenyl)-8-oxo-5,5a,6,8,8a,9-hexahydrofuro[3',4':6,7]naphtho[2,3-d][1,3]dioxol-5-yl-2-(dimethylamino)ethylcarbamate,

and their isomers, and also addition salts thereof with a pharmaceutically acceptable acid or base.

**9.** Process for the preparation of the compounds of formula **(I)**, **characterised in that** there is used as starting material a compound of formula **(II)** :

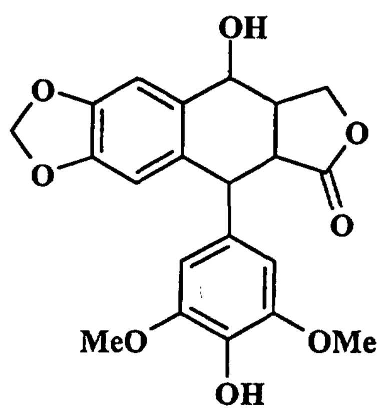

**(II)**

which is subjected under basic conditions :

- *either to the action of a compound of formula* ***(III)*** :

**$R'_1$ - X (III)**

wherein $R'_1$ represents a group selected from linear or branched ($C_1$-$C_6$)alkyl, aryl, aryl-($C_1$-$C_6$)alkyl in which the alkyl moiety may be linear or branched, heteroaryl, heteroaryl-($C_1$-$C_6$)alkyl in which the alkyl moiety may be linear or branched, linear or branched ($C_1$-$C_6$)alkylcarbonyl, arylcarbonyl, aryl-($C_1$-$C_6$)alkylcarbonyl in which the alkyl moiety may be linear or branched, heterocycloalkylcarbonyl, linear or branched ($C_1$-$C_6$)alkylsulfonyl, arylsulfonyl, and aryl-($C_1$-$C_6$)alkylsulfonyl in which the alkyl moiety may be linear or branched, and X represents a hydrogen atom or a halogen atom, to yield the compounds of formula **(IV/A)** :

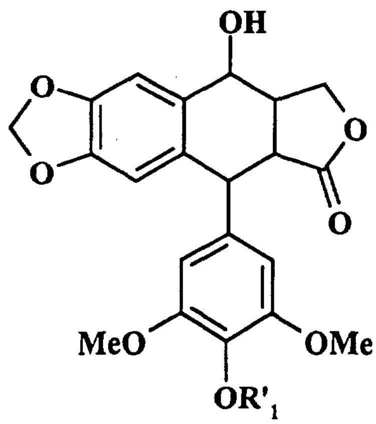

**(IV/A)**

wherein $R'_1$ is as defined hereinbefore,

- *or to the action of a compound of formula* ***(V)*** :

**G-L (V)**

wherein G represents a conventional protecting group for hydroxy functions and L represents a leaving group customary in organic chemistry,
to yield the compounds of formula **(IV/B)** :

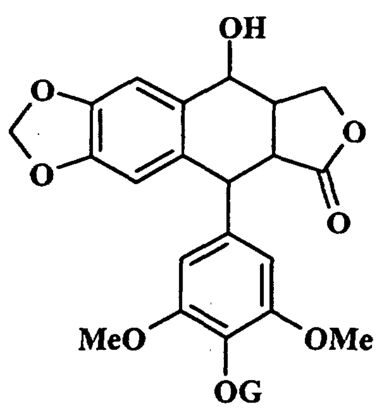

**(IV/B)**

wherein G is as defined hereinbefore,
the totality of the compounds of formulae (IV/A) and (IV/B) constituting the compounds of formula **(IV)**:

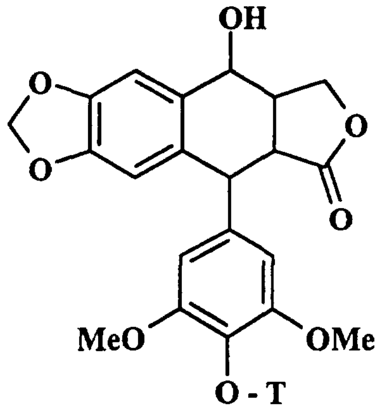

(IV)

wherein T represents a group $R'_1$ or G as defined hereinbefore,
which compound of formula (IV) is treated in basic medium with a compound of formula **(VI)** :

**Ph - O - C($R_2$)Cl** **(VI)**

wherein Ph represents an optionally substituted phenyl group and $R_2$ represents an oxygen atom or a sulphur atom, to yield the compounds of formula **(VII)** :

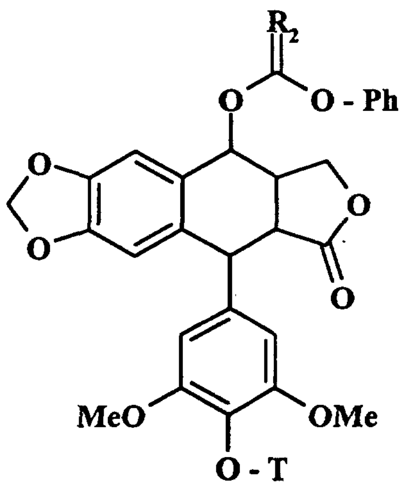

(VII)

wherein $R_4$, $R_3$ and X are as defined for formula (I),
to yield, respectively, the compounds of formula **(I/a)**, a particular case of the compounds of formula **(I)**, or the compounds of formula **(IX)**, according to whether T represents a group $R'_1$ or a group G,

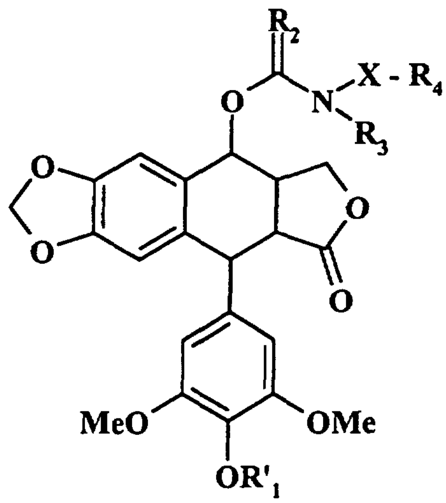

,

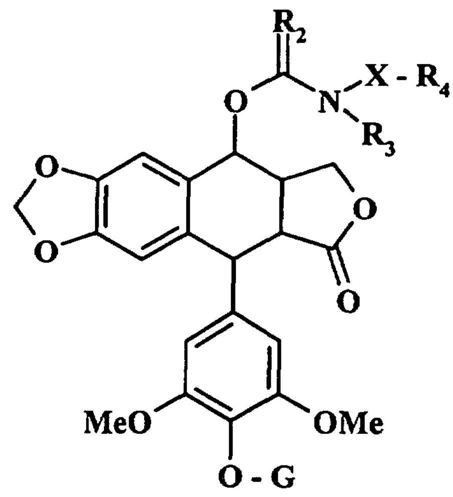

(I/a) (IX)

wherein R'$_1$, G, $R_2$, $R_3$, $R_4$ and X are as defined hereinbefore,
the hydroxy function of which compounds of formula (IX) is deprotected according to conventional methods of organic chemistry to yield the compounds of formula **(I/b)**, a particular case of the compounds of formula (I) :

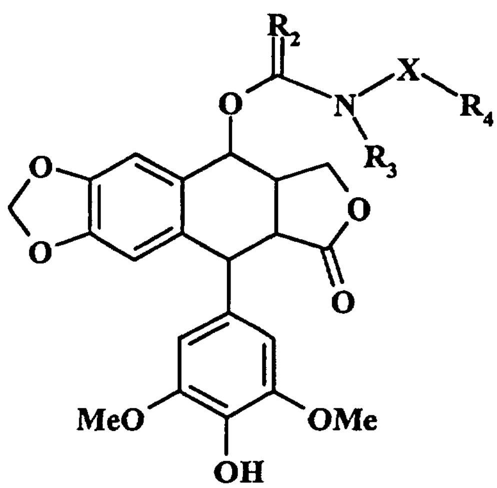

(I/b)

wherein $R_2$, $R_3$, $R_4$ and X are as defined for formula (I),
the compounds (I/a) and (I/b) constituting the totality of the compounds of the invention, which compounds are purified, if necessary, according to a conventional purification technique, may be separated, if desired, into their different isomers according to a conventional separation technique, and are converted, if desired, into their addition salts with a pharmaceutically acceptable acid or base.

**10.** Pharmaceutical compositions comprising as active ingredient at least one compound according to any one of claims 1 to 8, alone or in combination with one or more pharmaceutically acceptable, inert, non-toxic excipients or carriers.

**11.** Pharmaceutical compositions according to claim 10, containing at least one active ingredient according to any one of claims 1 to 8, for use in the treatment of cancers.

**Patentansprüche**

**1.** Verbindungen der Formel (I):

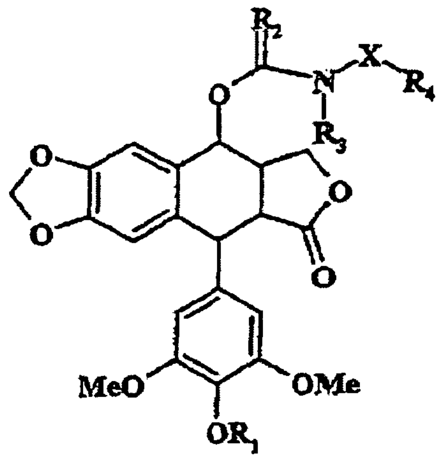

(I)

in der:

$R_1$ eine Gruppe ausgewählt aus Wasserstoff, geradkettigem oder verzweigtem ($C_1$-$C_6$)-Alkyl, Aryl, geradkettigem oder verzweigtem Aryl-($C_1$-$C_6$)-alkyl, Heteroaryl, geradkettigem oder verzweigtem Heteroaryl-($C_1$-$C_6$)-alkyl, geradkettigem oder verzweigtem ($C_1$-$C_6$)-Alkoxycarbonyl, Aryloxycarbonyl, geradkettigem oder verzweigtem Aryl-($C_1$-$C_6$)-alkoxycarbonyl, Heterocycloalkoxycarbonyl, geradkettigem oder verzweigtem ($C_1$-$C_6$)-Alkylsulfonyl, Arylsulfonyl, geradkettigem oder verzweigtem Aryl-($C_1$-$C_6$)-alkylsulfonyl und Phosphongruppen bedeutet,

$R_2$ ein Sauerstoffatom oder ein Schwefelatom darstellt,

$R_3$ ein Wasserstoffatom, eine geradkettige oder verzweigte ($C_1$-$C_6$)-Alkylgruppe, Cycloalkylgruppe oder geradkettige oder verzweigte Aryl-($C_1$-$C_6$)-alkylgruppe. bedeutet,

X eine geradkettige oder verzweigte ($C_1$-$C_6$)-Alkylengruppe bedeutet und

$R_4$ eine Gruppe ausgewählt aus:

- Amino, gegebenenfalls substituiert durch eine oder zwei gleichartige oder verschiedenartige Gruppen ausgewählt aus geradkettigem oder verzweigtem ($C_1$-$C_6$)-Alkyl, Aryl und geradkettigem oder verzweigtem Aryl-($C_1$-$C_6$)-alkyl,
- einer Gruppe der Formel -N($R_3$)-$X_1$-O$R_5$, in der $R_3$ die oben angegebenen Bedeutungen besitzt, $X_1$ eine geradkettige oder verzweigte ($C_1$-$C_6$)-Alkylengruppe darstellt und $R_5$ eine Gruppe bedeutet, ausgewählt aus Wasserstoff, geradkettigem oder verzweigtem ($C_1$-$C_6$)-Alkyl, Aryl und geradkettigem oder verzweigtem Aryl-($C_1$-$C_6$)-alkyl,
- einer Gruppe der Formel -N($R_3$)-$X_1$-N$R_6R_7$, in der $R_3$ und $X_1$ die oben angegebenen Bedeutungen besitzen und $R_6$ und $R_7$, die gleichartig oder verschieden sind, jeweils eine Gruppe bedeuten ausgewählt aus Wasserstoffatomen, geradkettigen oder verzweigten ($C_1$-$C_6$)-Alkylgruppen, Arylgruppen und geradkettigen oder verzweigten Aryl-($C_1$-$C_6$)-alkylgruppen,
- Hydroxy,
- geradkettigem oder verzweigtem ($C_1$-$C_6$)-Alkoxy,
- Aryloxy,
- einer Gruppe der Formel -O-$X_1$-O$R_5$, in der $R_5$ und $X_1$ die oben angegebenen Bedeutungen besitzen, und
- einer Gruppe der Formel -O-$X_1$-N$R_6R_7$, in der $R_6$, $R_7$ und $X_1$ die oben angegebenen Bedeutungen besitzen, bedeutet,

deren Isomere sowie deren Additionssalze mit einer pharmazeutisch annehmbaren Säure oder Base,
mit der Maßgabe, daß man:

- * *unter Aryl* eine Gruppe versteht ausgewählt aus Phenyl, Biphenyl, Naphthyl, Dihydronaphthyl, Tetrahydronaphthyl, Indenyl, Indanyl und Benzocyclobutyl, wobei jede dieser Gruppen gegebenenfalls eine oder zwei gleichartige oder verschiedenartige Substitutionen aufweist ausgewählt aus Halogen, Hydroxy, geradkettigem oder verzweigtem $(C_1-C_6)$-Alkyl, geradkettigem oder verzweigtem $(C_1-C_6)$-Alkoxy, Cyano, Nitro, Amino, geradkettigem oder verzweigtem $(C_1-C_6)$-Alkylamino, geradkettigem oder verzweigtem $(C_1-C_6)$-Dialkylamino, Carboxy, geradkettigem oder verzweigtem $(C_1-C_6)$-Alkoxycarbonyl, geradkettigem oder verzweigtem $(C_1-C_6)$ -Trihalogenalkyl, geradkettigem oder verzweigtem $(C_1-C_6)$-Alkylcarbonyloxy, geradkettigem oder verzweigtem $(C_1-C_6)$-Alkylcarbonyl und Aminocarbonyl, bei dem der Aminoteil gegebenenfalls durch eine oder zwei gleichartige oder verschiedenartige, geradkettige oder verzweigte $(C_1-C_6)$-Alkylgruppen substituiert ist,
- * *unter Heteroaryl* einen aromatischen Monocyclus, aromatischen Bicyclus oder Bicyclus versteht, bei dem einer der Ringe aromatisch und der andere teilweise hydriert ist, mit 5 bis 12 Kettengliedern, der in dem cyclischen System ein, zwei oder drei Heteroatome ausgewählt aus Sauerstoff, Stickstoff und Schwefel enthält, wobei der Heteroarylrest gegebenenfalls durch die gleichen Substituenten substituiert sein kann, wie sie oben für die Arylgruppe angegeben worden sind,
- * *unter Cycloalkyl* eine monocyclische oder bicyclische, gesättigte oder ungesättigte Gruppe versteht, die jedoch keinen aromatischen Charakter hat, und 3 bis 10 Kohlenstoffatome aufweist und gegebenenfalls durch eine oder mehrere, gleichartige oder verschiedenartige Gruppen substituiert ist, ausgewählt aus Halogen, geradkettigem oder verzweigtem $(C_1-C_6)$-Alkyl, geradkettigem oder verzweigtem $(C_1-C_6)$-Trihalogenalkyl, Hydroxy, Amino, geradkettigem oder verzweigtem $(C_1-C_6)$-Alkylamino und geradkettigem oder verzweigtem $(C_1-C_6)$ -Dialkylamino,
- * *unter Heterocycloalkyl* einen Cycloalkylrest versteht, wie er oben definiert worden ist, der in dem cyclischen System ein oder zwei Heteroatome ausgewählt aus Sauerstoff, Stickstoff und Schwefel aufweist, wobei der Heterocycloalkylrest gegebenenfalls durch einen oder mehrere Substituenten substituiert ist, wie sie oben bezüglich der Cycloalkylgruppe beschrieben worden sind,
- * *unter Isomeren* die Enantiomeren und Diastereoisomeren versteht.

**2.** Verbindungen der Formel (I) nach Anspruch 1, **dadurch gekennzeichnet, daß** $R_1$ ein Wasserstoffatom bedeutet, deren Isomere sowie deren Additionssalze mit einer pharmazeutisch annehmbaren Säure oder Base.

**3.** Verbindungen der Formel (I) nach Anspruch 1, **dadurch gekennzeichnet, daß** $R_2$ ein Sauerstoffatom bedeutet, deren Isomere sowie deren Additionssalze mit einer pharmazeutisch annehmbaren Säure oder Base.

**4.** Verbindungen der Formel (I) nach Anspruch 1, **dadurch gekennzeichnet, daß** X eine geradkettige $(C_2-C_4)$-Alkylengruppe darstellt, deren Isomere sowie deren Additionssalze mit einer pharmazeutisch annehmbaren Säure oder Base.

**5.** Verbindungen der Formel (I) nach Anspruch 1, **dadurch gekennzeichnet, daß** $R_3$ ein Wasserstoffatom oder eine geradkettige oder verzweigte $(C_1-C_6)$-Alkylgruppe bedeutet, deren Isomere sowie deren Additionssalze mit einer pharmazeutisch annehmbaren Säure oder Base.

**6.** Verbindungen der Formel (I) nach Anspruch 1, **dadurch gekennzeichnet, daß** $R_4$ eine Gruppe bedeutet ausgewählt aus:

- Amino, substituiert durch eine oder zwei gleichartige oder verschiedene, geradkettige oder verzweigte $(C_1-C_6)$ -Alkylgrupen,
- einer Gruppe der Formel $-N(R_3)-R_1-OR_5$, in der $R_3$ ein Wasserstoffatom oder eine geradkettige oder verzweigte $(C_1-C_6)$-Alkylgruppe darstellt und $X_1$ und $R_5$ die bezüglich der Formel (I) angegebenen Bedeutungen besitzen,
- und einer Gruppe der Formel $-O-X_1-OR_5$, in der $X_1$ und $R_5$ die bezüglich der Formel (I) angegebenen Bedeutungen besitzen, deren Isomere sowie deren Additionssalze mit einer pharmazeutisch annehmbaren Säure oder Base.

**7.** Verbindungen der Formel (I) nach einem der Ansprüche 1 oder 6, **dadurch gekennzeichnet, daß** $R_4$ eine Gruppe $-N(R_3)-X_1-OR_5$ oder $-O-X_1-OR_5$ darstellt, in der $R_3$ ein Wasserstoffatom, $X_1$ eine geradkettige oder verzweigte $(C_2-C_4)$-Alkylenkette und $R_5$ ein Wasserstoffatom bedeuten, deren Isomere sowie deren Additionssalze mit einer pharmazeutisch annehmbaren Säure oder Base.

**8.** Verbindungen der Formel (I) nach Anspruch 1, nämlich:

- (5*S*,5a*S*,8a*S*,9*R*)-9-(4-Hydroxy-3,4-dimethoxyphenyl)-8-oxo-5,5a,6,8,8a,9-hexahydrofuro[3',4':6,7]naphtho [2,3-*d*][1,3]dioxol-5-yl-2-(dimethylamino)-ethyl(methyl)-carbamat,
- (5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho [2,3-*d*][1,3]dioxol-5-yl-2-(dimethylamino)-ethyl-(methyl)-carbamat,
- (5*S*,5a*S*,8a*S*,9*R*)-9-(4-Hydroxy-3,5-dimethoxyphenyl)-8-oxo-5,5a,6,8,8a,9-hexahydrofuro[3',4':6,7]naphtho [2,3-*d*][1,3]dioxol-5-yl-3-(dimethylamino)-propylcarbamat,
- (5*S*,5a*S*,8a*S*,9*R*)-9-(4-Hydroxy-3,5-dimethoxyphenyl)-8-oxo-5,5a,6,8,8a,9-hexahydrofuro[3',4':6,7]naphtho [2,3-*d*][1,3]dioxol-5-yl-2-(2-hydroxyethoxy)-ethylcarbamat,
- (5*S*,5a*S*,8a*S*,9*R*)-9-(4-Hydroxy-3,5-dimethoxyphenyl)-8-oxo-5,5a,6,8,8a,9-hexahydrofuro[3',4':6,7]naphtho [2,3-*d*][1,3]dioxol-5-yl-2-[(2-hydroxyethyl)-amino]-ethylcarbamat und
- (5*S*,5a*S*,8a*S*,9*R*)-9-(4-Hydroxy-3,5-dimethoxyphenyl)-8-oxo-5,5a,6,8,8a,9-hexahydrofuro[3',4':6,7]naphtho [2,3-*d*][1,3]dioxol-5-yl-2-(dimethylamino)-ethylcarbamat

sowie deren Isomere und deren Additionssalze mit einer pharmazeutisch annehmbaren Säure oder Base.

**9.** Verfahren zur Herstellung der Verbindungen der Formel (I), **dadurch gekennzeichnet, daß** man als Ausgangsprodukt eine Verbindung der Formel **(II)** verwendet:

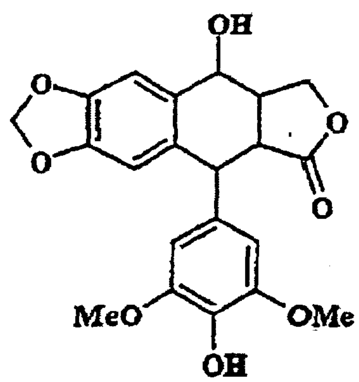

**(II)**

welche man unter basischen Bedingungen:

- *entweder der Einwirkung einer Verbindung der Formel **(III)** unterwirft:*

$$R'_1 - X \qquad \textbf{(III)}$$

in der $R'_1$ eine Gruppe bedeutet, ausgewählt aus geradkettigem oder verzweigtem ($C_1$-$C_6$)-Alkyl, Aryl, geradkettigem oder verzweigtem Aryl-($C_1$-$C_6$)-alkyl, Heteroaryl, geradkettigem oder verzweigtem Heteroaryl-($C_1$-$C_6$)-alkyl, geradkettigem oder verzweigtem ($C_1$-$C_6$)-Alkylcarbonyl, Arylcarbonyl, geradkettigem oder verzweigtem Aryl-($C_1$-$C_6$)-alkylcarbonyl, Heterocycloalkylcarbonyl, geradkettigem oder verzweigtem ($C_1$-$C_6$)-Alkylsulfonyl, Arylsulfonyl und geradkettigem oder verzweigtem Aryl-($C_1$-$C_6$)-alkylsulfonyl, und X ein Wasserstoffatom oder ein Halogenatom bedeuten, zur Bildung der Verbindungen der Formel **(IV/A)**:

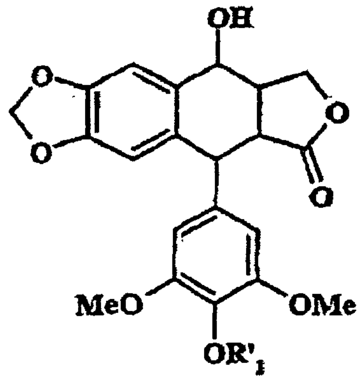

**(IV/A)**

in der $R'_1$ die oben angegebenen Bedeutungen besitzt,

- *oder der Einwirkung einer Verbindung der Formel (V) unterwirft:*

**G - L (V)**

in der G eine klassische Schutzgruppe für Hydroxy-Funktionen darstellt, und L eine in der organischen Chemie übliche austretende Gruppe bedeutet,
zur Bildung der Verbindungen der Formel **(IV/B)**:

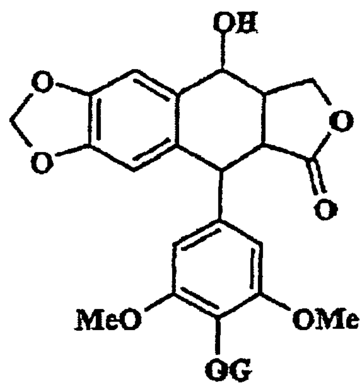

**(IV/B)**

in der G die oben angegebenen Bedeutungen besitzt,
wobei die Gesamtheit der Verbindungen der Formeln (IV/A) und (IV/B) die Verbindungen der Formel **(IV)** bildet:

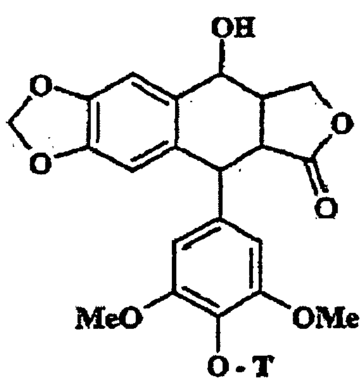

**(IV)**

in der T eine Gruppe $R'_1$ oder G, wie sie oben definiert worden sind, darstellt,

welche Verbindung der Formel (IV) man in basischem Medium mit einer Verbindung der Formel **(VI)** behandelt:

**Ph - O - C($R_2$)Cl (VI)**

in der Ph eine gegebenenfalls substituierte Phenylgruppe und $R_2$ ein Sauerstoffatom oder ein Schwefelatom bedeuten,
zur Bildung der Verbindungen der Formel **(VII)**:

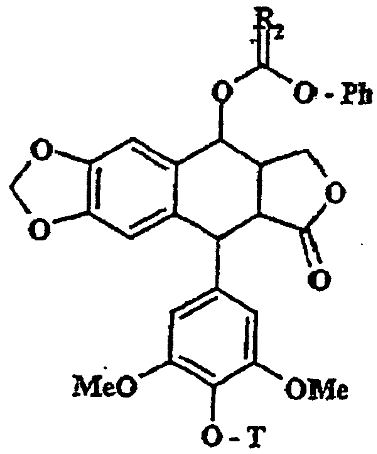

**(VII)**

in der T, Ph und $R_2$ die oben angegebenen Bedeutungen besitzen, welche Verbindung der Formel (VII) man in basischem Medium mit einer Verbindung der Formel **(VIII)** umsetzt:

**$R_4$ - X - NH - $R_3$ (VIII)**

in der $R_4$, $R_3$ und X die bezüglich der Formel (I) angegebenen Bedeutungen besitzen,
so daß man in Abhängigkeit davon, ob T eine Gruppe $R'_1$ oder G bedeutet, die Verbindungen der Formel **(I/a)**, einem Sonderfall der Verbindungen der Formel (I) oder der Formel **(IX)** erhält:

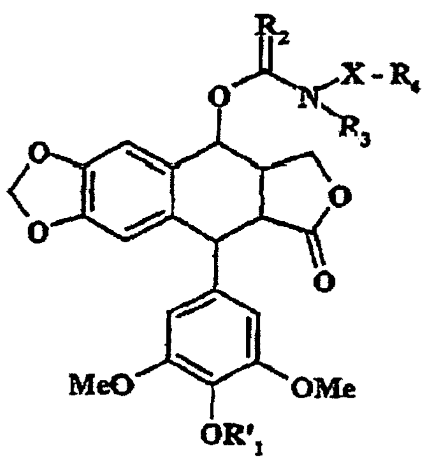

**(I/a)**

,

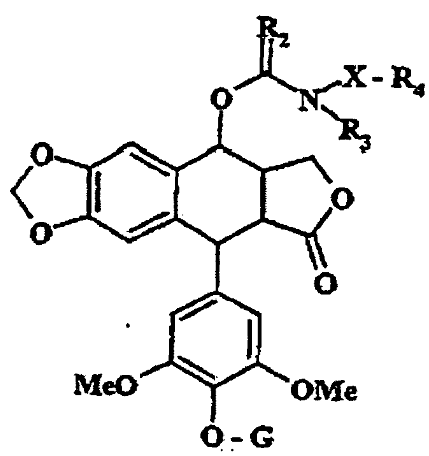

**(IX)**

in denen $R'_1$, G, $R_2$, $R_3$, $R_4$ und X die oben angegebenen Bedeutungen besitzen, von welchen Verbindungen der Formel (IX) man die Schutzgruppe der Hydroxyfunktion mit Hilfe klassischer Methoden der organischen Chemie abspaltet zur Bildung der Verbindungen der Formel **(I/b)**, einem Sonderfall der Verbindungen der Formel (I):

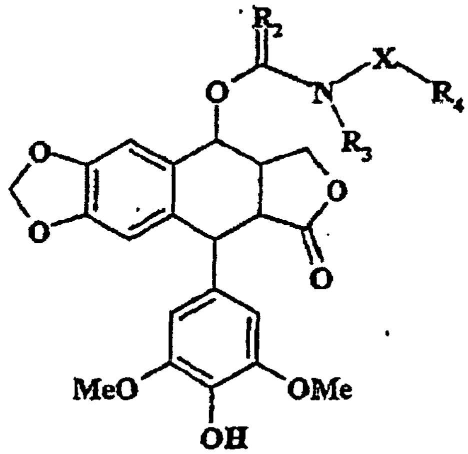

(I/b)

in der $R_2$, $R_3$, $R_4$ und X die bezüglich der Formel (I) angegebenen Bedeutungen besitzen,
welche Verbindungen der Formeln (I/a) und (I/b), welche die Gesamtheit der erfindungsgemäßen Verbindungen bilden, man gegebenenfalls mit Hilfe einer klassischen Reinigungsmethoden reinigt, welche gewünschtenfalls mit Hilfe einer klassischen Trennungsmethode in ihre verschiedenen Isomeren getrennt werden können und welche man gewünschtenfalls in ihre Additionssalze mit einer pharmazeutisch annehmbaren Säure oder Base umwandelt.

**10.** Pharmazeutische Zubereitungen enthaltend als Wirkstoff mindestens eine Verbindung nach einem der Ansprüche 1 bis 8 allein oder in Kombination mit einem oder mehreren inerten, nichttoxischen, pharmazeutisch annehmbaren Trägermaterialien oder Bindemitteln.

**11.** Pharmazeutische Zubereitungen nach Anspruch 10, enthaltend mindestens einen Wirkstoff nach einem der Ansprüche 1 bis 8 zur Behandlung von Krebs.